# EUROPEAN PATENT APPLICATION

(11) **EP 0 913 692 A1**
(43) Date of publication of application: **06.05.1999**
(21) Application number: 97119018.6
(22) Date of filing: 31.10.1997
(51) Int. Cl.: G01N 33/68, C07K 14/78, C07K 16/18

(54) **An immunoassay for procollagen-III-C-terminal propeptide**

(71) Applicant: BAYER AG, 51368 Leverkusen (DE)
(72) Inventor: Burchardt, Elmar R., Dr., 42113 Wuppertal (DE); Kroll, Werner, Dr., 42659 Solingen (DE); Neumann, Rainer, Dr., 50937 Köln (DE); Schröder, Werner, Dr., 42113 Wuppertal (DE)

(57) **Abstract**

The present invention relates an immunoassay for procollagen-III-C-terminal-propeptide which comprises antibodies that specifically bind to the protein in a human fluid sample as well as the generation of recombinant PIIICp and deletion constructs for the generation of above mentioned antibodies.

## Description

The present invention relates an immunoassay for procollagen-III-C-terminal-propeptide which comprises antibodies that specifically bind to the protein in a human fluid sample as well as the generation of recombinant PIIICP and deletion constructs for the generation of above mentioned antibodies.

### PIIICP: cDNA and amino acid sequence and structure

The C-terminal procollagen (III) propeptide (PIIICP) molecule is a proteolytic fragment emanating from the specific cleavage of procollagen (III) by C-proteinase after exocytosis.

Collagens I and III are synthesized as prepropeptides and are extensively modified posttranslationally. Among the specific intracellular modifications are glycosylations, enzymatic hydroxylation reactions involving lysine and proline in its 3- and 4-positions, and the specific proteolytic removal of the leader peptide. The modified propeptides spontaneously assemble into [α₁(III)]₃ homotrimers in the case of collagen (III), and mostly [α₁(I)]α₂ (I) heterotrimers as well as - to a lesser extent - [α₁(I)]₃ homotrimers in the case of collagen (I) in the microsomal compartments. After exocytosis, the propeptide is first cleaved at the C-terminus of the nascent collagen and then at the N-terminus by a set of specific endoproteases. Recently, the enzyme responsible for the cleavage of the C-terminal extension peptide in collagens I, II, and III has been identified at the gene level (Kessler *et al*., 1996).

It has been firmly established that the nucleation of triple helix formation in collagens I and III starts from the C-terminal region of the procollagen molecules and proceeds in a more or less "zipper-like" fashion towards the N-terminus (Engel and Prockop, 1991). The molecular interactions governing the association of PIIIP to form the a₁(III) homotrimer are so far poorly understood.

PIIICP occurs as a trimer consisting of three identical monomeric PIIICP subunits that are linked by intermolecular disulfide bridges (Dion and Myers, 1987). Structural considerations and site-directed mutagenesis experiments with a collagen minigene (Lees and Bulleid, 1994) have led to the conclusion that at least 4 and probably 6 cysteine residues are involved in intramolecular disulfide bridge formation and that only cysteines 51 and 68 are involved in intermolecular disulfide bridge formation. It has been observed, however, that the region around this intermolecular cystine bridge is critical for the correct formation of intramolecular disulfide bridges and that a Cys→Ser mutation in that region also leads to impaired intramolecular disulfide bridge formation. On the other hand it has been observed that the trimerization of collagen (III) fibrils can proceed even when interchain cystine bridge formation has become impossible by Cys→Ser mutations in position Cys51 (relative to the C-proteinase cleavage site) (Lees and Bulleid, 1994).

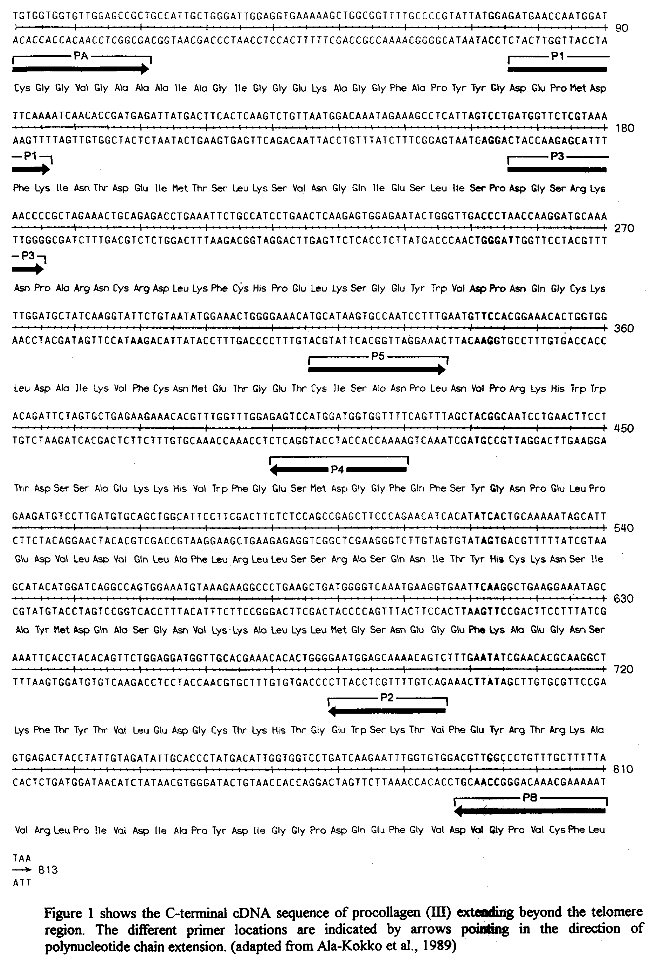

The PIIICP cDNA sequence has been published and two entries are found in the Genbank data base [Accession #'s X14420 (Ala-Kokko *et al*., 1989) and X01742 (Loidl *et al*., 1984)] (figure 1). The sequence was identical to the sequence published (figure 1) by Ala-Kokko *et al*., 1989, and deviated from the sequence published by Loidl *et al*. (1984) in one amino acid.

### Antibodies against PIIICP

There is only sparse information on antibodies generated against PIIICP. One source (Minz and Mann, 1990) reports the generation of antibodies against the arbitrarily chosen oligopeptide fragment
**N-DEPMDFKINTDEIMTSLKSA-C**
which corresponds to the region immediately C-terminal to the C-proteinase cleavage site. Both polyclonal rabbit antibodies and murine monoclonal antibodies were described and characterized. The specificity of the antibodies was checked by testing for crossreactivity with the C-terminal type I procollagen propeptide (PICP) before and after cleavage by C-proteinase and in the reduced as well as unreduced form. The monoclonal and polyclonal antibodies were specific for type III procollagen. However, the sensitivity of the monoclonal antibody was extremely low and only 10 µg quantities of purified reduced and unreduced PIIICP could be stained when the monoclonal antibody was used for Western blot detection while 1 µg quantities were not stained. In addition, the monoclonal antibody showed cross-reactivity with PICP under reducing conditions.

A secondary structure prediction of the amino acid sequence of PIIICP performed with the DNASTAR program on a Hewlett Packard Vectra XM with a 5/133 processor revealed that the choice of this sequence may have been suboptimal for the purpose of generating antibodies that sensitively recognize intact PIIICP in patient sera. the sequence is not very hydrophilic and has a relatively low surface probability in the context of the entire protein as well as a relatively low calculated antigenicity.

### Measurements of collagen fragments

Collagen (III) is the characteristic collagen of parenchymal organs and is the second most prevalent collagen in fibrotic tissues. Although collagen (I) is the most prevalent scar-forming collagen and although collagen (I) is upregulated even more drastically in liver fibrosis than collagen (III) it occurs in large amounts in bone (Uitto *et al*., 1986) and is therefore of limited value in the differential diagnosis of fibrotic processes in parenchymal organs. C-terminal procollagen (I) propeptide serum determinations have therefore primarily been used for the monitoring of disorders of bone metabolism (Eriksen *et al*., 1993).

Serum assays for the determination of collagen (III) fragment concentrations, particularly for PIIINP, are already in use for the monitoring of fibrotic diseases. Elevated circulating PIIINP levels may, however, also originate from the cleavage of deposited collagen (III) and thus reflect fibrolysis rather than fibrogenesis (Schuppan, 1991). This is due to the fact that PIIINP is present on the surface of collagen (III) after its deposition in the extracellular matrix (Fleischmajer *et al*., 1981). The molecular masses of the PIIINP species emanating from fibrolysis seem to be distinct from the species generated in fibrogenesis and include higher as well as lower molecular mass species that circulate in the plasma (Niemelä *et al*., 1982). Although PIIINP serum determinations are fairly well established for the monitoring of liver fibrosis in various underlying diseases such as primary biliary cirrhosis (e.g. Davis and Madri, 1987), chronic hepatitis B and C (Murawaki *et al*., 1995), and alcoholic liver disease (Savolainen *et al*., 1984), they are of little help in the establishment of the diagnosis. This is due to two different characteristics of PIIINP serum determinations: 1. Circulating PIIINP levels seem to correlate with the acute phase of liver inflammation when excessive collagen deposition is not yet visible histologically and may in fact never become manifest (Savolainen *et al*.; 1984, Hansen *et al*., 1995) 2. Increased circulating PIIINP levels in clinical settings can indicate fibrolysis rather than fibrogenesis because uncleaved PIIINP is present on the surface of collagen (III) fibers and is liberated in the process of collagen degradation (Davis and Madri, 1987).

In contrast to PIIINP, PIIICP is rapidly cleaved from nascent collagen (III) fibrils and does not remain associated with deposited collagen (Dion and Myers, 1987). Experimental evidence suggests that fiber assembly cannot properly proceed without cleavage of the C-terminal propeptide and that the assembly of larger diameter fibers is inhibited as long as the C-terminal propeptide is still associated with the tropocollagen fibrils (Miyahara *et al*., 1984). Only upon the proteolytic cleavage of procollagen by C-proteinase is the larger fiber assembly initiated by this entropy-driven process (Kadler *et al*., 1987). Because of the necessity to cleave the C-terminal propeptide for the nascent collagen fiber to grow, determinations of PIIICP should be more specific for the purpose of monitoring collagen synthesis rather than collagen degradation when compared to the presently available PIIINP assays. PIIICP serum levels thus have the potential to emerge as a new specific serum marker of fibrogenesis.

### Immunoassay applications:

The immunoassay of the invention comprises reaction of at least two antibodies with a human fluid sample, wherein at least one of the antibodies specifically binds to region I and one antibody to region II. Preferably the antibodies are monoclonal antibodies and both of said two antibodies of the assay specifically bind to the protein.

The antibody or antibodies of the assay that specifically bind to the procollagen-III-C-terminal-propeptide preferably exhibit less than about 3% cross-reactivity, in an assay such as that described in Example 6 below or similar assay with a human plasma sample, with PIIINP, PICP, collagen III, collagen I and collagen VI.

"Antibody", "antibody of the invention" or other similar term as used herein includes a whole immunoglobulin as well as antigenic binding fragments or immunoreactive fragments which specifically bind to the procollagen-III-C-terminal-propeptide, including Fab, Fab', F(ab')₂ and F(v).

The human fluid sample used in the assay of the invention can be any sample that contains the procollagen-III-C-terminal-propeptide, e.g. blood or urine. Typically a serum or plasma sample is employed.

Antibodies of the invention can be prepared by techniques generally known in the art, and are typically generated to a sample of a procollagen-III-C-terminal-propeptide. The antibodies also can be generated from an immunogenic peptide that comprises one or more epitopes of the procollagen-III-C-terminal-propeptide that are exhibited by native procollagen-III-C-terminal-propeptide.

More particularly, antibodies can be prepared by immunizing a mammal with a purified sample of a procollagen-III-C-terminal-propeptide, or an immunogenic peptide as discussed above, alone or complexed with a capture. Suitable mammals include typical laboratory animals such as sheep, goats, rabbits, guinea pigs, rats and mice. Rats and mice, especially mice, are preferred for obtaining monoclonal antibodies. The antigen can be administered to the mammal by any of a number of suitable routes such as subcutaneous, intraperitoneal, intravenous, intramuscular or intracutaneous injection.

Preferably immunization is done by subcutaneous, intraperitoneal, or intravenous injection. The optimal immunizing interval, immunizing dose, etc. can vary within relatively wide ranges and can be determined empirically based on this disclosure. Typical procedures involve injection of the antigen several times over a number of weeks. Antibodies are collected from serum of the immunized animal by standard techniques and screened to find antibodies specific for the procollagen-III-C-terminal-propeptide. Monoclonal antibodies can be produced in cells which produce antibodies and those cells used to generate monoclonal antibodies by using standard fusion techniques for forming Hybridoma cells. Typically this involves fusing an antibody producing cell with an immortal cell line such as a myeloma cell to produce the hybrid cell. Alternatively, monoclonal antibodies can be produced from cells by the method of Huse *et al*. (1989).

One suitable protocol provides for intraperitoneal immunization of a mouse with a composition comprising purified procollagen-III-C-terminal-propeptide conducted over a period of about two to seven months. Spleen cells then can be removed from the immunized mouse. Sera from the immunized mouse is assayed for titers of antibodies specific for procollagen-III-C-terminal-propeptide prior to excision of spleen cells. The excised mouse spleen cells are then fused to an appropriate homogenic or heterogenic (preferably homogenic) lymphoid cell line having a marker such as hypoxanthine-guanine phosphoribosyltransferase deficiency (HGPRT) or thymidine kinase deficiency (TK). Preferably a myeloma cell is employed as the lymphoid cell line. Myeloma cells and spleen cells are mixed together, e.g. at a ration of about 1 to 4 myeloma cells to spleen cells. The cells can be fused by the polyethylene glycol (PEG) method. The thus cloned hypridoma is grown in a culture medium, e.g. RPMI-1640 (Moore *et al*., 1967).

Hybridomas, grown after the fusion procedure, are screened such as by radioimmunoassay or enzyme immunoassay for secretion of antibodies that bind specifically to the procollagen-III-C-terminal-propeptide, e.g. antibodies are selected that bind to the complete procollagen-III-C-terminal-propeptide, to region I of the subsequence but not to region II. Preferably an ELISA is employed for the screen. Hybridomas that show positive results upon such screening can be expanded and cloned by limiting dilution method. Further screens are preferably performed to select antibodies that can bind to procollagen-III-C-terrninal-propeptide in solution as well as in human fluid samples.

The assay of the invention is illustrated by the following protocol using alkaline phosphatase as the label of the conjugate antibody. A test sample, e.g. a human serum sample, is added to a procollagen-III-C-terminal-propeptide antibody (i.e., an antibody that binds to the procollagen-III-C-terminal-propeptide) bound on a capture such a microtiter plate and the antibody-antigen reaction is conducted, followed by addition of the alkaline phosphatase labeled procollagen-III-C-terminal-propeptide antibody conjugate obtained as outlined above, and then a further antibody-antigen reaction conducted. The antibodies are typically dissolved in solution prior to contact with a test sample. Suitable diluents include those known in the art for use in immunoassays. A specifically preferred solution for dissolving the antibodies for contact with a test sample contains 20 mµ Tris, 500 mµ sodium chloride, 0.05 mg/ml mouse IgG and 5% BSA (iv/v)..

Preferably, both the capture and conjugate antibodies of the assay of the invention specifically bind to the procollagen-III-C-terminal-propeptide, and the antibody bound to the support, the human fluid sample and labeled antibody are incubated together, followed by a single wash step to remove any unreacted labeled antibody and the human plasma sample other than the reacted procollagen-III-C-terminal-propeptide. Suitable washing agents include those known in the art for use in immunoassays. A specifically preferred washing buffer solution contains 27.2 g/l imidazole, 17.5 g/l sodium chloride and 4 ml/l Tween 20. If necessary a substrate for alkaline phosphatase is added to the assay and the reaction products are assayed for enzyme activity by measuring the absorbance or fluorescence of the resulting substance. The detected amount of bound labeled antibody is directly proportional to the concentration of procollagen-III-C-terminal-propeptide in the assayed human serum sample. Thus a quantitative determination of the procollagen-III-C-terminal-propeptide concentration in the plasma test sample can be determined by comparison of the absorbance or fluorescence of the test sample with absorbance values obtained from standardized solutions that contain known amounts of procollagen-III-C-terminal-propeptide. It may be desirable to prepare calibration curves from absorbance values obtained from a number of standardized solutions to facilitate interpretation of values obtained from a test sample.

A specifically preferred immunoassay of the invention was conducted as follows. The capture monoclonal antibody and conjugate monoclonal antibody labeled with horseradish alkaline phosphatase (AP) was incubated together with a human plasma sample at 37°C for 30 minutes. The plate was then washed and incubated for 15 minutes at room temperature with AP substrate and the bound conjugate was quantitated.

Procollagen-III-C-terminal-propeptide cannot be purified from a human serum sample by use of capture complexes coupled with one or more antibodies of the invention in sufficient quantities for immunization although limited amounts were purified and hence the invention includes methods for obtaining purified procollagen-III-C-terminal-propeptide using the antibodies of the invention ad related apparatus. A suitable purification procedure provides coupling an antibody of the invention on an appropriate capture as is known in the art, such as a gel or resin, then packing the capture in a column, and then eluting a sample solution containing procollagen-III-C-terminal-propeptide through the column to selectively adsorb the procollagen-III-C-terminal-propeptide. The antibody can be suitably coupled onto the capture by known methods, e.g. the cyanogen bromide method and glutaraldehyde method, aqueous carbodiimide method, active ester method, and the like. The antibody also may be physically adsorbed on the surface of the capture.

### Recombinant production of PIIICP

A number of publications have reported the recombinant production of procollagens. One publication (Lee *et al*., 1990) claims the production of collagen a₂(I) mutation in the C-proteinase cleavage site in A2 cells derived from the rat liver epithelial cell line W8 which is deficient for collagen a₂(I). Two recent publications report the recombinant expression of collagen a₁(III) minigenes (Lees and Bulleid, 1994; Bulleid *et al*., 1996). The sole recombinant expression of the C-terminal procollagen a₁(III) propeptide, e.g. for the purposes of selective immunizations, or the expression of truncated PIIICP, e.g. for the purpose of epitope-mapping, has not been reported. Immunizations have only been caried out with arbitrarily chosen synthetic peptides of uninvestigated secondary structure (Minz and Mann, 1990).

### Monitoring fibrotic disease processes

A diverse array of diseases is associated with the inappropriate or unregulated production of collagen. PIIICP measurements can be performed from patient sera or other body fluids from patients with these diseases. Among these are liver fibrosis of various etiologies, alcoholic cirrhosis, biliary cirrhosis, hepatitis, schistosomiasis, cardiac fibrosis of various etiologies, idiopathic interstitial fibrosis, idiopathic pulmonary fibrosis, interstitial pulmonary fibrosis, acute pulmonary fibrosis, acute respiratory distress syndrome, perimuscular fibrosis, pericentral fibrosis, dermatofibroma, kidney fibrosis, diabetic nephropathy, glomerulonephritis, systemic and localized scleroderma, keloids, hypertrophic scars, severe joint adhesions, arthrosis, myelofibrosis, corneal scaring, cystic fibrosis, muscular fibrosis, Duchenne's muscular dystrophy, esophageal stricture, retroabdominal scaring, Crohn's disease, ulcerative colitis, aneurysms of large vessels.

Further fibrotic disorders can be induced or initiated by scar revisions, plastic surgeries, glaucoma, cataract fibrosis, corneal scaring, joint adhesions, graft vs. host disease, tendon surgery, nerve entrapment, dupuytren's contracture, OB/GYN adhesions, pelvic adhesions, peridural fibrosis, diseases of the thyroid gland or the parathyroids, metastatic bone disease, multiple myeloma, and restenosis.

Early diagnosis is essential for a potential treatment of these diseases. Up to the present, serum tests for fibrotic diseases are not well established and the final diagnosis must entirely rely on invasive biopsies.

PIIICP measurements can also be performed to measure the rate of collagen synthesis in patients undergoing therapy with glucocorticosteroids.

Furthermore, the antibodies directed against PIIICP can be used to assess collagen synthesis in tissue samples from patients with fibrotic disease by immunohistochemical staining of kryostat and paraffine sections.

### Examples

### Example: Production of the recombinant PIIICP

### Cloning of PIIICP cDNA

The entire cDNA sequence encoding PIIICP was PCR-cloned from a human aorta cDNA library (QUICK-Clone cDNA, Clontech, USA), subcloned into the bacterial expression vector pQE30 (Qiagen, Germany) and sequence-verified. Sequence information was obtained from the DNASTAR program [Genbank Accession #'s X14420 (Ala-Kokko *et al*., 1989) and X01742 (Loidl *et al*., 1984)] (figure 1).

PCR was carried out following the manufacturer's recommendations for buffer conditions with 2.5 units of *Taq* polymerase (Boehringer Mannheim, Germany). Briefly, two specific primers with *Bam*HI and *Hin*dIII restriction sites integrated at their respective 5' ends (PA and PB, table 1) were added to a final concentration of 500 fM each. 1 µl of QUICK-Clone cDNA (Clontech, USA) was used as a template in a 100 µl PCR reaction. PCR was carried out with a high performance UNO thermocycler manufactured by Biometra, Germany. The following program was used: initial template denaturation step: 5 min at 94°C. Standard cycle conditions were: 45 sec at 94°C, 60 sec at 55°C, 60 sec at 72°C. 30 Cycles were carried out. A 420 sec extension step completed the program. The resulting PCR fragment was purified and phosphorylated. The bacterial expression vector pQE30 (Qiagen, Germany) was compatibly digested with *Bam*HI and *Hin*dIII, and the linearized plasmid was dephosphorylated. The enzymatically modified PCR fragment and the vector were ligated and the ligation products were transformed into the *E*. *coli* strain sure 2 (Stratagene, USA). A correct clone (PIIICP4.1) was identified. All cloning procedures were performed according to the procedures described in Sambrook *et al*., 1989, using enzymes from Boehringer Mannheim, Germany. The clone was expanded, and the sequence of the new plasmid verified by two-directional nucleotide sequencing with an Applied Biosystems (USA) sequencer.

The sequence was identical to the sequence published (figure 1) by Ala-Kokko *et al*., 1989, and deviated from the sequence published by Loidl *et al*. (1984) in one amino acid.

### Expression and purification of the recombinant N-terminal His tag PIIICP fusion protein and of N- and C-terminally truncated mutants

Briefly, after a second transformation, the corresponding N-terminal His tag fusion protein (table 1) was expressed in the *E. coli* strain M15 carrying the pREP4 plasmid (according to QIAexpressionist manual, p. 35, Qiagen, Germany, incubation temperature 37°C). The recombinant fusion protein was purified over a Ni-NTA Superflow column (Qiagen, Germany) according to the purification protocol from the manufacturer (Protocol 7, QIAexpressionist manual, pp. 45-46, Qiagen, Germany).

Four truncated mutants of the PIIICP molecule were generated. The plasmid PIIICP4.1 served as a template for the four truncated constructs described below. The four additional constructs encompass the entire coding sequence of the procollagen (III) propeptide region except for the first 30 (PIIICP2.1.7) or 90 (PIIICP5.1) codons 3 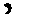 to the C-proteinase cleavage site and the 38 (PIIICP1.1) or 130 (PIIICP5.1) most 3 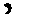 -located codons of the coding sequence of the propeptide, respectively.

As an example for the generation of the truncated PIIICP mutants the generation of the C-terminal deletion mutant PIIICP1.1 will be described in more detail. For the generation of this 3 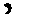 end-truncated mutant, 1 µg of the PIIICP4.1 plasmid was used as the template and the amplification was carried out with primers P1 and P2 (see table 2 for sequence information). P1 and P2 contained *Bam*HI and *Hin*dIII restriction sites at their ends, respectively. 12 PCR cycles were carried out under the same cycle conditions as described above. The resulting PCR product was subcloned into the bacterial expression vector pQE30 employing a procedure completely analogous to the generation of PIIICP4.1.

A plasmid encoding the entire procollagen (III) propeptide cDNA sequence except for the 38 most 3 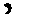 -located codons of the PIIICP coding sequence for procollagen (III) propeptide was obtained and was designated PIIICP1.1.

The generation of the plasmids PIIICP2.1.7, PIIICP5.1 and 6.1 was performed in analogy to the procedure described for the generation of PIIICP1.1.

The expression and purification of the recombinant N-terminal His tag PIIICP fusion proteins lacking the 30 or 90 most N-terminal and the 38 or 130 most C-terminal amino acids, respectively, was performed as described above for the untruncated protein. Table 1 summarizes the calculated molecular weights of the recombinant proteins.

All proteins could be expressed in the M15 strain at approximate yields exceeding 20 mg/l (figure 3). Most of the protein was located in inclusion bodies. The truncated mutants were expressed at slightly higher levels than the protein encoded by the full length cDNA. The protein was liberated from the Ni-NTA column upon elution with buffer E (Protocol 7, QIAexpressionist manual, pp. 45-46, Qiagen, Germany).

### Amino acid sequencing of the purified recombinant proteins

Sample preparation of PIIICP to remove buffer and other contaminants was performed using the ProSpin sample preparation cartridge from Applied Biosystems (US) the Centricon concentrators (Amicon, USA), by blotting onto PVDF-membrane after SDS-gel electrophoresis or by gel filtration in formic acid on a silica gel column. A vertical electrophoresis unit (LKB/Pharmacia, Germany), a trans blot cell and a power supply for blotting model 250/2.5 (BioRad, Germany) were used. Reagents for electrophoresis and PVDF-membrane were from BioRad. All other chemicals used were of pro analysis or biochemical grade (Merck, Germany). The prestained protein marker was from BRL (Germany).

N-terminal sequence analyses of collagen PIIICP-fragments were performed using the gas-liquid-solid-phase protein sequencer 473A from Applied Biosystems. The standard sequencer program was used. The sequencer, the different running programs, the cycles, as well as the PTH-separation system, are described in detail in the respective manual (User's manual protein sequencing system model 473A (1989), Applied Biosystems, USA). The detection of PTH-amino acids were performed on-line using a RP-18-column (220 mm x 2 mm, 5 µ-material) PTH-column from Applied Biosystems. The PTH-amino acids were identified and quantified by a 50 pM standard of all PTH-amino acids. The data were collected and integrated using the sequencer data system 610A from Applied Biosystems. About 20 ng of the corresponding PIIICP fragments was used for sequencing.

The PIIICP4.1, PIIICP1.1, PIIICP2.1.7, PIIICP5.1, and PIIICP6.1 proteins were N-terminally sequenced as described above. The number of cycles was sufficient in each case to reach the collagen sequence of the fusion protein. Table 3 summarizes the results from the amino acid sequencing of three representative PIIICP proteins. It was identical to the published sequence in each case.

### Multimerization attempts

The purified proteins were separated by electrophoresis on a 12.5% SDS gel (Sambrook *et al*., 1989). To determine which molecular weight disulfide-linked PIIICP species and truncated mutants were recombinantly expressed in the *E. coli* strain M15 with the pREP4 plasmid, mercaptoethaol (5% (v/v)) as the reducing agent was omitted from the sample buffers and the resulting bands were compared with a molecular size standard ad with their mercaptoethanol-reduced counterparts.

Simultaneously, the thioredoxin-deficient *E. coli* strain DMB4 (Derman *et al.*, 1993) was transformed with the PIIICP4.1 plasmid. Recombinant protein production was initiated as described above, the only exception being that the incubation took place at room temperature in accordance with the Derman group's suggestions. The induced recombinant PIIICP from this strain was subsequently purified and analyzed as described for the M15-derived protein.

By comparison of the unreduced PIIICP species with the reduced protein, that appeared as one band on an SDS gel, it became apparent that the recombinant PIIICP was synthesized as several different molecular weight- and electrophoretic mobility species in the *E. coli* strain M15 harboring the pREP4 plasmid. Thus, the observed diversity is likely due to differences in disulfide crosslinking. The monomers were the predominant species in all cases but higher molecular weight bands possibly representing dimeric PIIICP and PIIICP trimers were also discernable. Notably, several bands probably representing different disulfide-linked monomeric PIIICP species with different secondary structures were visible, especially with the truncated PIIICP protein PIIICP2.1.7 that lacked the 30 most N-terminally located amino acids and - to a lesser extent - with the truncated PIIICP1.1 protein that had a deletion of the 38 most C-terminal amino acids. Multimerization was also observed with the PIIICP4.1 expression product that encompasses the entire PIIICP amino acid sequence and 25 amino acids N-terminal of it. For the most part, the PIIICP4.1 expression product appeared as a single monomeric species under non-reducing conditions but exhibited a slightly increased electrophoretic mobility when compared to the reduced form. In addition, two additional higher molecular weight species, likely presenting dimers and trimers, were also discernable.

A pronounced difference in electrophoretic mobility between the expression in the E. *coli* strains M15 with the pREP4 plasmid and the thioredoxin-deficient strain DMB4 was not observed (results not shown).

Further attempts to trimerize the monomeric protein by various renaturation attempts did not lead to enhanced multimer formation (results not shown).

The multimerization experiments are important to answer the question if the epitopes present in the *E. coli* protein are sufficiently similar to the eukaryotic expression product probably circulating human plasma.

For the first time, we have been able to investigate recombinant human PIIICP association *ex vivo* with pure material obtained from *E. coli*, an organism that lacks the capacity to specifically hydroxylate prolyl or lysyl residues in collagen-like molecules.

Our results indicate that PIIICP is predominantly synthesized as a monomeric protein under these conditions. It seems however, that even unmodified PIIICP has the inherent ability to form multimers. In our experiments we have demonstrated the occurence of higher molecular weight species exhibiting electrophoretic mobilities, probably including trimer and dimer formation, that are surprisingly similar to those observed in the formation of procollagen trimers in a cell free eukaryotic *in vitro* translation system as described recently (Bulleid *et al.*, 1996).

It is interesting to note that the trimerization process in the eukaryotic *in vitro* translation system was markedly dependent on prolyl hydroxylation. It has been observed that the trimerization of collagen (III) fibrils can proceed even when interchain cystine bridge formation has become impossible by Cys→Ser mutations in position Cys51 (relative to the C-proteinase cleavage site) (Lees and Bulleid, 1994). The deletion of the telopeptidic cysteine present in PIIICP4.1 as in PIIICP2.1.7, but not in PIIICP1.1, does not result in less pronounced multimerization. This finding indicates that the telopeptidic cystine bridge is not essential for trimer formation and is in line with previous reports (Bulleid *et al*., 1996).

An impairing effect on trimerization was observed when prolyl hydroxylation was inhibited by the addition of α,α 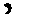 -dipyridyl. The authors draw the conclusion that the trimerization need not necessarily proceed via nucleation from the C-propeptide but can also proceed by direct triple helix formation or from the N-terminal propeptide and that prolyl hydroxylation is essential for the proper trimerization.

The results obtained with recombinant PIIICP and its truncated mutants from the expression in *E. coli* point in a different direction. As *E. coli* is uncapable of hydroxylating proline residues in collagen-like molecules and as higher molecular weight species probably including trimers have been observed with all three expression products, it is likely that the capacity to form trimers is intrinsically present in unhydroxylated PIIICP species but is less pronounced than in hydroxylated species. It is possible that the high degree of conservation regarding the number and position of cysteins across species borders observed with PIIICP (Dion and Myers, 1987) is due to an intrinsic tendency of the protein to self-assemble. Hydroxylation probably assists in this process.

From these considerations it is possible to draw conclusions about the antigenic resemblence of the epitopes in recombinant *E. coli* PIIICP versus the correctly processed human protein. Our results indicate that the material that we used for immunizations was similar to the unhydroxylated precursors encountered in the cell-free eukaryotic *in vitro* translation system. According to our results, the propensity to form trimers appears to be an inherent property of PIIICP and the correct assembly of the trimeric protein probably encompasses several trimeric intermediate precursor states that are possibly similar to the *E. coli* protein in terms of the three-dimensional structure. The unhydroxylated protein is not only identical to the human trimer in terms of primary sequence but it may also resemble many aspects of its secondary and tertiary structure. This surprising fact increased the chances to identify antibodies directed against the recombinant *E. coli* material that specifically recognize the human antigen as well. This may hold true even if the majority of the recombinant material occurs in a monomeric form, as IgG's can potentially bind to trimers with an apparently higher affinity than to monomers if one antibody can simultaneously bind to two identical epitopes on adjacent chains (Rohde *et al*., 1983).

### Example 2: Immunization

BALB/c female mice were immunized with procollagen-III-C-terminal-propeptide to provide spleen donors for the PEG fusion, disclosed below, that generated monoclonal antibodies that specifically bind to the procollagen-III-C-terminal-propeptide.

BALB/c female mice were sensitized with 10 µg each of purified procollagen-III-C-terminal-propeptide in immunogen emulsion prepared as follows:
0.125 ml Procollagen-III-C-terminal-propeptide 325 µg/ml in 50 mM tris/50 mM NaCl/0.1 mµ EDTA pH 7.4)
1.500 ml Complete Freund's Adjuvant
1.375 ml Dulbecco's Phosphate Buffered Saline

Each mouse was injected with 0.5 ml of this immunogen emulsion i.p. The mice were boosted with doses of up to 50 µg of purified procollagen-III-C-terminal-propeptide i.p. in an immunization protocol that was carried out for approximately six months.

### Example 3: ELISA Assay for procollagen-III-C-terminal-propeptide Antibodies

### Coating Microtiter plates with procollagen-III-C-terminal-propeptide

Procollagen-III-C-terminal-propeptide was diluted in a coating buffer (carbonate buffer pH9) at a concentration of 1 µg/ml. 100 µl of each solution (10 ng procollagen-III-C-terminal-propeptide) was placed in each well of the microtiter plates, which were sealed and incubated overnight at 2-8°C. The contents of the wells were then aspirated and the plates were washed once with wash/storage buffer, the wash aspirated, and the plates again resealed. The plates were stored at 1-8°C until use.

### Example 4: Preparation of Hybridomas

Hybridomas secreting monoclonal antibodies to procollagen-III-C-terminal-propeptide were generated by two cell fusions. The PEG fusion technique was employed. The myeloma cells used were HRPT-minus P3 - X63-Ag8.653 (P3X) (ATCC CRL1580). Selection for hybrids was accomplished using HAT media (hypoxanthine, aminopterin and thymidine), unfused P3X myeloma cells will not survive in this medium as they lack the apparatus to utilize hypoxanthine to produce purines and the aminopterin present in the medium blocks endogenous synthesis of purines and pyrimidines.

### Fusion to form Hybridoma procollagen-III-C-terminal-propeptide

### Splenocyte preparation

Spleen cells were obtained from a mouse immunized with procollagen-III-C-terminal-propeptide as described for Example 1 above. The cells were teased from the spleen using a forceps and needle, then suspended in 12 ml of cold 20% Complete Medium without serum (RPMI 1640 base, Gibco). The cells were then centrifuged 200 g for 10 minutes after which the supernatant was removed by aspiration, and the cells were resuspended again in cold medium. This washing process was repeated twice, and the cells resuspended in a final volume of 10 ml. The viable cell count of the splenocytes was 1.7 x 10⁸ at a viability of 98% by Trypan Blue exclusion technique. Counting methods employed were as disclosed by M. Absher, 1973. In brief, a volume of cell suspension was mixed with an equal volume of 0.02 % Trypan Blue and the cells were counted in a hemocytometer by standard counting methods.

### Myeloma preparation

The myeloma cells were harvested mechanically, pooled, and centrifuged at 200 g for 10 minutes after which the supernatant was removed by aspiration, an the cells were resuspended in 50 ml of 20% Complete Medium. The viable cell count of the myeloma cells was 2.9 x 10⁶ viable cells per ml at a viability of 76%.

### Fusion of the splenocytes and myeloma cells

The splenocytes and 15 ml of the myeloma cell suspension were combined at a spleen cell to myeloma cell ratio of approximately 4:1 with a total viable cell count of 2.13 x 10⁸. The volume was brought up to 50 ml with cold 20% Complete Medium without serum and the cells then centrifuged at 200 g for 10 minutes. The cell pellet was then washed twice with this medium at a volume of 50 ml. After the final wash, the supematant was removed by aspiration and the pellet centrifuged at 200 g for 3 minutes and the remaining supernatant aspirated. The cells were then fused with 40% PEG (molecular weight 7,000 to 9,000 Da) buffered in RPMI 1640; fusion was performed in a tube held in a warm (37°C) water bath. 1 ml of PEG solution warmed to 37°C was added to the pellet and incubated for 1 minute. The PEG solution was then diluted by addition of 20 ml of warm 20% Complete Medium without serum. The fused cells were incubated at 37°C for 10 minutes and then centrifuged at 200 g for 10 minutes.

The fused cell pellet was then resuspended in 50 ml of 20% Complete Medium and plated at cell densities of 1.09 x 10⁵ to 4.26 x 10⁵ per well. The cells were plated in a final volume of 200 µl per well of 20% Complete Medium with 250 units/ml IL-6. After overnight incubation at 37°C and 10% CO₂, one half of the medium in each well was aspirated and the cells were fed with 20% HAT, 20% HAT with 5 µg/ml STM or 20% HAT with 500 units/ml IL-6. The cells were visually scanned and fed periodically with these media for several weeks, while the growth of the hybridomas was monitored and growing wells screened for the presence of anti-procollagen-III-C-terminal-propeptide antibodies beginning at day 12 to 14 post-fusion.

### Example 5: Application of recombinant PIIICP and the corresponding truncated mutants for the characterization of the epitope-specificity of monoclonal antibodies raised against recombinant PIIICP

Monoclonal antibodies were raised and preliminarily tested for epitope-specificity. To screen for epitope-specific monoclonal antibodies, all three recombinant proteins were used.
Those monoclonal antibodies recognizing all five recombinant proteins were directed against either the N-terminal His tag or the middle region between the 90 most N-terminal and the 130 most C-terminal amino acids of the procollagen (III) propeptide. To distinguish these two binding epitopes an unrelated control protein (4.5.2) with an N-terminal His tag fusion sequence identical to the PIIICP expression products was used. Those antibodies recognizing the 4.5.2 antigen were directed against the His tag sequence.

Antibodies recognizing only the protein derived from clone PIIICP4.1 were classified as specific for the region N-terminal of the C-Proteinase cleavage site and were not used any further as well.

All monoclonal antibodies recognizing the complete propeptide (PIIICP4.1-derived) as well as the C-terminally truncated proteins (PIIICP1.1- and PIIICP5.1-derived) while not reacting with the N-terminally truncated proteins (PIIICP2.1.7- or PIIICP6.1-derived) were classified as epitope-specific for the 30 or 90 most N-terminal amino acids of the propeptide, respectively.

An analogous approach was used to determine the binding epitope specificity of antibodies recognizing C-terminal regions of PIIICP.

To characterize the binding characteristics of the newly generated monoclonal antibodies, both ELISA and Western Blot techniques were applied. Two suitable antibodies that recognized complementary epitopes and that allowed the very sensitve detection of native PIIICP from patient samples were selected (48B14 and 48D19)
a.) ELISA: After identification of the optimal titer using complete recombinant PIIICP in an ELISA assay where the recombinant PIIICP was coated at a fixed mass concentration, the deletion proteins were coated at the same concentration, and the signal intensity was determined in exact analogy to the complete PIIICP (derived from PIIICP4.1). Table 4 summarizes the results for this ELISA assay for the two monoclonal antibodies 48B14 and 48D19 with the different antigens.
b.) Western Blot: After determination of the optimal liters of the different antibodies, all three PIIICP variants were electrophorized in approximately equal molar concentrations and Western Blots were performed. Differences in antigen recognition could thus be correlated with epitope specificity.

### Example 6: Establishment of a PIIICP immunoassay based on the recognition of two different epitopes on the PIIICP molecule by two different monoclonal antibodies

### Sandwich Assay Using Monoclonal Antibodies

Measurements of the C-terminal procollagen (III) propeptide (PIIICP) concentrations in patients' sera were carried out employing the sandwich technology. An epitope-specific monoclonal antibody recognizing an N-terminal epitope of the PIIICP molecule specifically bound and immobilized the circulating serum PIIICP. A second antibody directed against C-terminal parts of PIIICP was used to detect this complex.

Monoclonal 48B14 was immobilized on a solid capture of microtiter wells. A sample of patient plasma containing procollagen-III-C-terminal-propeptide and a measured amount of monoclonal 48D19, covalently labeled with an enzyme, specifically horseradish alkaline phosphatase, were then added to the immobilized 48B14 for 30 minutes at 37°C. The unreacted conjugate (48B14) and any unreacted procollagen-III-C-terminal-propeptide are then removed by washing. The amount of bound labeled antibody is directly proportional to the concentration of procollagen-III-C-terminal-propeptide in the test sample.

The combination of two site-specific monoclonal antibodies as described above, exclusively recognized higher molecular weight species of PIIICP that are related to the *de novo* deposition of procollagen (III) in the extracellular matrix in the sera of all patients and controls. This specificity for intact PIIICP further distinguishes the novel assay from assays that additionally recognize lower molecular weight species. Shorter PIIICP fragments can represent degradation products emanating from PIIICP and may not necessarily reflect recent collagen synthesis. Therefore, the new assay described above is very specific for the purpose of monitoring collagen synthesis.

### Example 7: PIIICP Assay on an automated immunoanalyzer

The PIIICP assay was set up as a sandwich immunoassay with simultaneous addition of both antibody reagents and sample, and late addition of magnetic particles.
- 10 µl serum sample and 10 µl of magnetic particle buffer were pipetted into the reaction cuvette.
- 30 sec. later 65 µl reagent R1 and 65 µl reagent R2 were dispensed into the cuvette. Reagent R1 contained a fluoresceinated monoclonal anti-pIIICP antibody in a buffered solution. Reagent R2 contained a monoclonal anti-pIIICP antibody conjugated to alkaline phosphatase in a buffered solution.
- The reaction mixture was incubated for 21 min at 37°C to form the sandwich immune complex.
- 10µl of magnetic particles coated with an anti-fluorescein antibody were added, and the mixture was incubated at 37°C for additional 8 min.
- The immune complex bound to the particles was separated from the reaction mixture by applying an external magnetic field. The particles were washed to remove excess sample and reagent.
- 300 µl p-nitrophenolate were added to the reaction mixture. The colored p-nitrophenolate anion was formed, and the rate of formation was directly proportional to the PIIICP amount present in the sample. At low concentrations the rate of absorbance increase was monitored at 405 nm, at high color formation rate the filter wavelength was switched to 450 nm.

### Example 8: Use of recombinant PIIICP as a substrate to measure the catalytic activity of C-proteinase

For the determination of catalytic activity of C-proteinase an ELISA assay was established, using recombinant N-terminal 6xHis PIIICP as substrate. For immobilization of the substrate Ni-NTA HisSorb Strips (Qiagen, Hilden, Germany) were used.

To establish the C-proteinase activity assay a simple ELISA assay was performed to allow for the immobilization and detection of the PIIICP. To this end, recombinant 6xHis-tagged PIIICP extending 25 amino acids N-terminal of the C-proteinase cleavage site (PIIICP4.1) was diluted in 0.2% (w/v) BSA/PBS, applied to the wells and incubated for 1 h. After washing with 0.25% (v/v) Tween 20/PBS, a peroxidase labeled monoclonal PIIICP-specific antibody (diluted in 1% (w/v) BSA/PBS) was added at a suitable concentration. Using a peroxidase substrate a highly sensitive and specific detection of the immobilized PIIICP was possible in a concentration-dependent fashion.

Using some variations of this format a determination of catalytic activity of C-proteinase was possible: After immobilization of the substrate to the Ni-NTA HisSorb Strips and washing with C-proteinase buffer (50 mM Tris, 100 mM NaCl, pH 8.0) C-proteinase was added and incubated for 3 hours. After extensive washing with PBS the remaining PIIICP-substrate was now detected. The details of the experimental procedures were analogous to the ELISA format described above. Comparing the absorption of the C-proteinase-containing wells with the negative controls (no C-proteinase) a significant decrease of PIIICP as detected with an antibody specific for a region C-terminal of the C-proteinase cleavage site was measured. This decrease was found to correspond to the amount of cleaved PIIICP. By contrast, the analogous N-terminal deletion protein PIIICP2.1.7 that lacks the C-proteinase cleavage sequence was still detected in unaltered amounts after incubation with C-proteinase and was therefore not cleaved.

### Example 9: Use of recombinant PIIICP as a marker to control the therapeutic efficacy of C-proteinase inhibitors from patient sera

The new serum PIIICP immunoassay can be used to monitor the effects of a therapy with inhibitors of procollagen C-proteinase or of BMP-1 or of isoforms or splice variants of these two proteins. While the concentration of intact PIIICP diminishes corresponding to the inhibition of C-Proteinase, unstable uncleaved procollagen (III) is deposited and serum parameters of extracellular matrix breakdown are typically upregulated.

### Example 10: Measurement of PIIICP concentrations in patients with classified fibrotic liver disease

Measurements of the C-terminal procollagen (III) propeptide (PIIICP) concentrations in patients' sera were carried out employing the technology explained in example 7.

In a first approach sera from 65 healthy blood donors of different ages and both sexes were screened with this assay. pIIICP was below the detection-limit in all of these sera. The range of pIIICP concentration was between -0.2 and 0.0 ng/ml.

In a second step sera from patients with various liver diseases were tested with this assay. The results are shown in the following table:

| Sera # | Diagnosis | pIIICP (Ng/ml) |
|---|---|---|
| LF 1454 | Hepatitis C infection | 5.5 ng/ml |
| LF 1462 | Hepatitis C infection | 3.5 ng/ml |
| LF 1856 | PBC | 8.0 ng/ml |
| LF 1861 | PBC | 8.6 ng/ml |
| LF 1862 | PBC | 7.6 ng/ml |
| LF 1863 | PBC | 8.3 ng/ml |
| LF 1866 | Alcohol induced Liverdisease | 13.9 ng/ml |
| LF 1870 | Alcohol induced Liverdisease | 15.4 ng/ml |
| LF 1873 | Alcohol induced Liverdisease | 14.9 ng/ml |
| LF 1876 | HBV infection | 7.2 ng/ml |
| LF 1877 | HBV infection | 6.9 ng/ml |
| LF 1878 | HBV infection | 6.3 ng/ml |
| LF 1879 | HBV infection | 7.4 ng/ml |
| LF 1880 | HBV infection | 7.2 ng/ml |

From these data it is deduced that measuring pIIICP level in sera of patients is a useful marker to differentiate between various types of liver-disease.

### Example 11: Immunohistochemical staining of tissue sections from patient samples

In order to detect the cells producing cIIICP paraffin and cryostatsections from liver-biopsies and from dermatological sections were processed according to routine histological protocols after incubation with a pIIICP alkaline phosphatase labelled antibody. As substrate Fast-Red solution was applied to the slides and cells producing pIIICP are stained red after development.

Two examples are given here.
Case I 1476-85/97 is a scleroderma case classified as DSS. In this case cells in all parts of the section are producing pIIICP.
Case I 822-31/97 is a section from the liver of a patient with livercirrhosis. In the high magnification presented here only a limited number of cells are producing pIIICP.

From other experiments not presented here we know that these cells are identical to that one which can be stained by an anti-SMA antibody. We therefore conclude that these cells are ITO cells.

### Literature

Absher M (1973) Hemocyameten counting. in Tissue culture: methods and application. edited by Kruse PF and Patterson MK, Academic Press
Ala-Kokko L, Kontusaari S, Baldwin CT, Kuivaniemi H, Prockop DJ (1989) Structure of cDNA clones coding for the entire preproα₁(III) chain of human type III procollagen. Biochem. J. 260: 509-516
Bulleid NJ, Wilson R, Lees JF (1996) Type-III procollagen assembly in semi-intact cells: chain association, nucleation and triple-helix folding do not require formation of inter-chain disulfide bonds but triple-helix nucleation does require hydroxylation. Biochem. J. 317: 195-202
Davis BH and Madri JA (1987) An immunohistochemical and serum ELISA study of type I and III procollagen aminopeptides in primary biliary cirrhosis. Am. J. Path. 128: 265-275
Derman AI, Prinz WA, Belin D, Beckwith J (1993) Mutations that allow disulfide bond formation in the cytoplasm of *Escherichia coli.* Science 262: 1744-1747
Dion DS and Myers JC (1987) COOH-terminal propeptides of the major human procollagens. J. Mol. Biol. 193: 127-143
Engel J and Prockop DJ (1991) The zipper-like folding of collagen triple helices and the effects of mutations that disrupt the zipper. Ann. Rev. Biophys. Biophys. Chem. 20: 137-152
Eriksen EF, Charles P, Melsen F, Mosekilde L, Risteli L, Risteli J (1993) Serum markers of type I collagen formation and degradation in metabolic bone disease: Correlations to bone histomorphometry. J. Bone Miner. Res. 8: 127-132
Fleischmajer R, Timpl R, Tudermann L, Raisher L, Wiestner M, Perlish JS, Graves PN (1981) Ultrastructural identification of extension aminopropeptides of type I and III collagens in human skin. Proc. Natl. Acad. Sci. USA 78: 7360-7364
Hansen M, Stoltenberg M, Host NB, Boesby S, Lorenzen I, Bentsen KD (1995) Glucocorticoids inhibit the synthesis rate of type III collagen, but do not affect the hepatic clearance of its aminoterminal propetide (PIIINP). Scand. J. Lab. Invest. 55: 543-548
Huse WD, Sastry L, Iverson SA, Kang AS, Alting-Mees M, Burton DR, Benkovic SJ, Lerner RA (1989) Generation of a large combinatorial library of the immunoglobulin repertoire in phage lambda. Science 246: 1275-1281
Kadler KE, Hojima Y, Prockop DJ (1987) Assembly of collagen fibrils *de novo* by cleavage of the type I pC-collagen with procollagen C-proteinase. Assay of critical concentration demonstrates that collagen self-assembly is a classical example of an entropy-driven process. J. Biol. Chem. 260: 15696-15701
Kessler E, Takahara K, Biniaminow L, Brusel M, Greenspan DS (1996) Bone morphogenetic protein-1: The type I procollagen C-proteinase. Science 271: 360-362
Lee ST, Kessler E, Greenspan DS (1990) Analysis of site-directed mutations in human pro-α₂(I) collagen which block cleavage by the C-proteinase. J. Biol. Chem. 265: 21992-21996
Lees JF and Bulleid NJ (1994) The role of cysteine residues in the folding and association of the COOH-terminal propeptide of types I and III procollagen. J. Biol. Chem. 269: 24354-24360
Loidl HR, Brinker JM, May M, Pihalajaniemi T, Morrow S, Rosenbloom J, Myers JC (1984) Molecular cloning and carboxy-propeptide analysis of human type III procollagen. Nucl. Acids Res. 12: 9383-9394
Mintz KP and Mann KG (1990) Detection of procollagen biosynthesis using peptide-specific antibodies. Matrix 10: 186-199
Miyahara M, Hayashi K, Berger J, Tanzawa K, Njieha FK, Trelstad RL, Prockop DJ (1984) Formation of collagen fibrils by enzymatic cleavage of precursors of type I collagen *in vitro.* J. Biol. Chem. 259: 9891-9898
Moore GE, Gerner RE, Franklin HA (1967) Culture of normal human leukocytes. JAMA 199: 87-92
Murawaki Y, Ikuta Y, Nishimura Y, Koda M, Kawasaki H (1995) Serum markers for connective tissue turnover in patients with chronic hepatitis B and chronic hepatitis C: A comparative analysis. J. Hepatol. 23: 145-152
Niemelä O, Risteli L, Sotaniemi EA, Risteli J (1982) Heterogeneity of the antigens related to the aminoterminal propeptide of type III procollagen in human serum Clinica Chimica Acta 124: 39-44
Parfitt AM, Simon LS, Villanueva AR, Krane SM (1987) Procollagen type I carboxy-terminal extension peptide in serum as a marker collagen biosynthesis in bone. Correlation with iliac bone formation rates and comparison with total alkaline phosphatase. J. Bone Miner. Res. 2: 427-436
Rohde H, Bruckner P, Timpl R (1983) Immunochemical properties of the aminopropeptide of procollagen type III. Eur. J. Biochem. 135: 197-202
Sambrook J, Fritsch EF, Maniatis T (1989) Molecular Cloning. A Laboratory Manual. 2nd edition. Cold Spring Harbor Laboratory Press.
Savolainen ER, Goldberg B, Leo MA, Velez M, Lieber CS (1984) Diagnostic value of serum procollagen peptide measurements in alcoholic liver disease. Alcohol. Clin. Exp. Res. 8: 384-389
Schuppan D (1991) Connective tissue polypeptides in serum as parameters to monitor antifibrotic treatment in hepatic fibrogenesis. J. Hepatology 13 (Suppl. 3): S17-S25
Uitto J, Murray LW, Blumberg B, Shamban A (1986) Biochemistry of collagen in diseases. Ann. Int. Med. 105: 740-756

**Table 1**

| **Summary of the calculated molecular weights of the proteins** | | |
|---|---|---|
| Designation | Molecular weight (only collagen sequence) [kD] | Molecular weight (His tag fusion protein) [kD] |
| PIIICP4.1 | 29.694 | 31.317 |
| PIIICP1.1 | 23.260 | 24.883 |
| PIIICP2.1.7 | 24.166 | 25.785 |
| PIIICP5.1 | 13.037 | 14.659 |
| PIIICP6.1 | 18.474 | 20.097 |

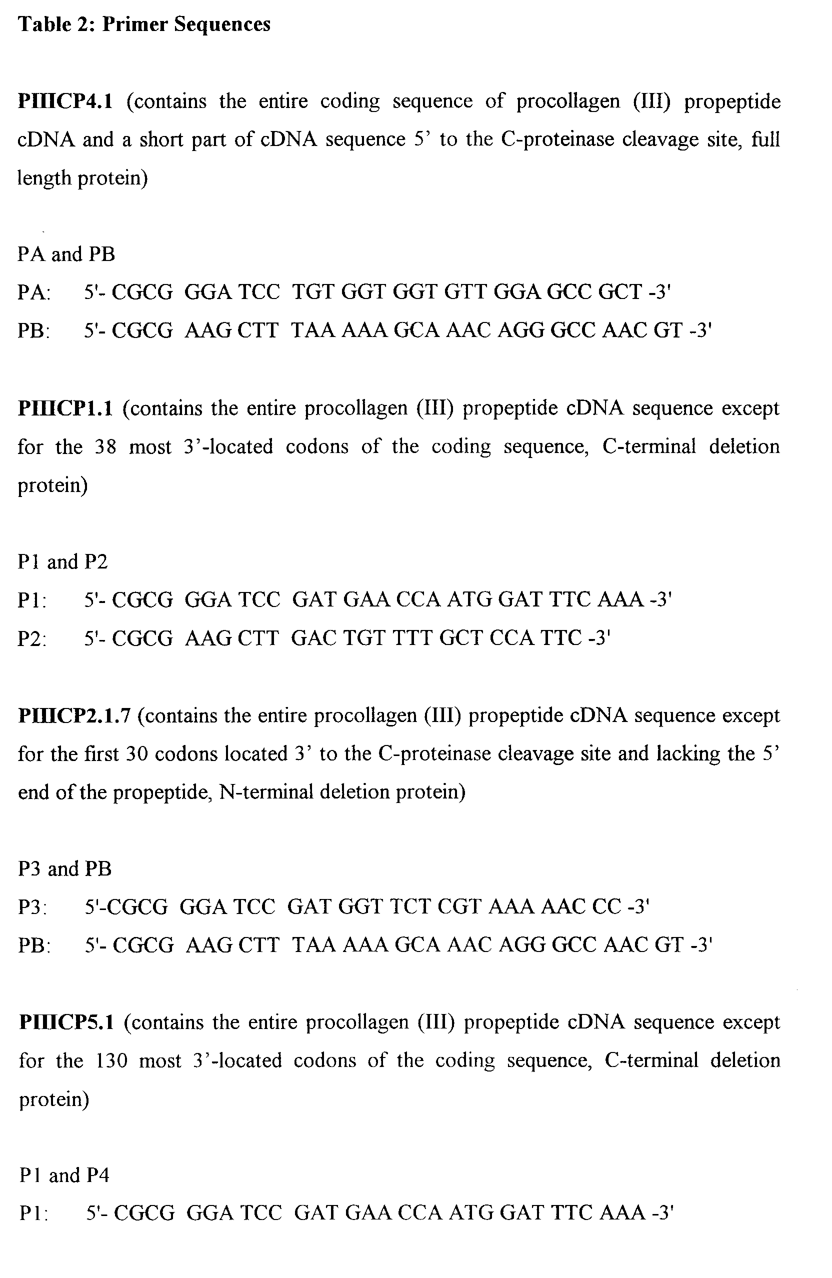

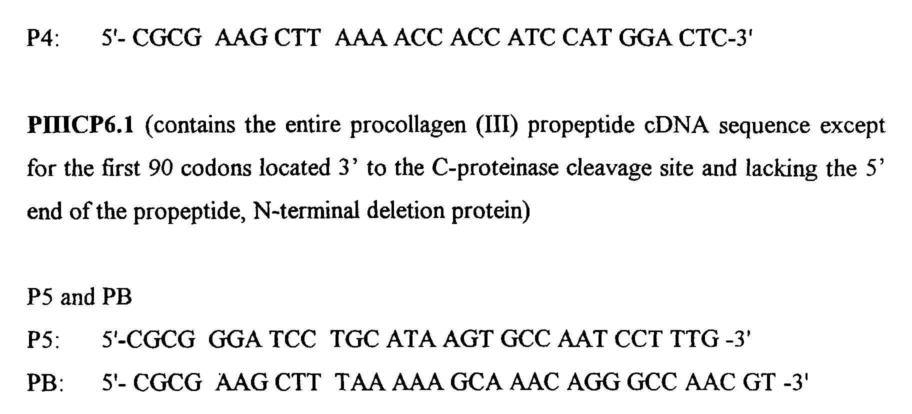

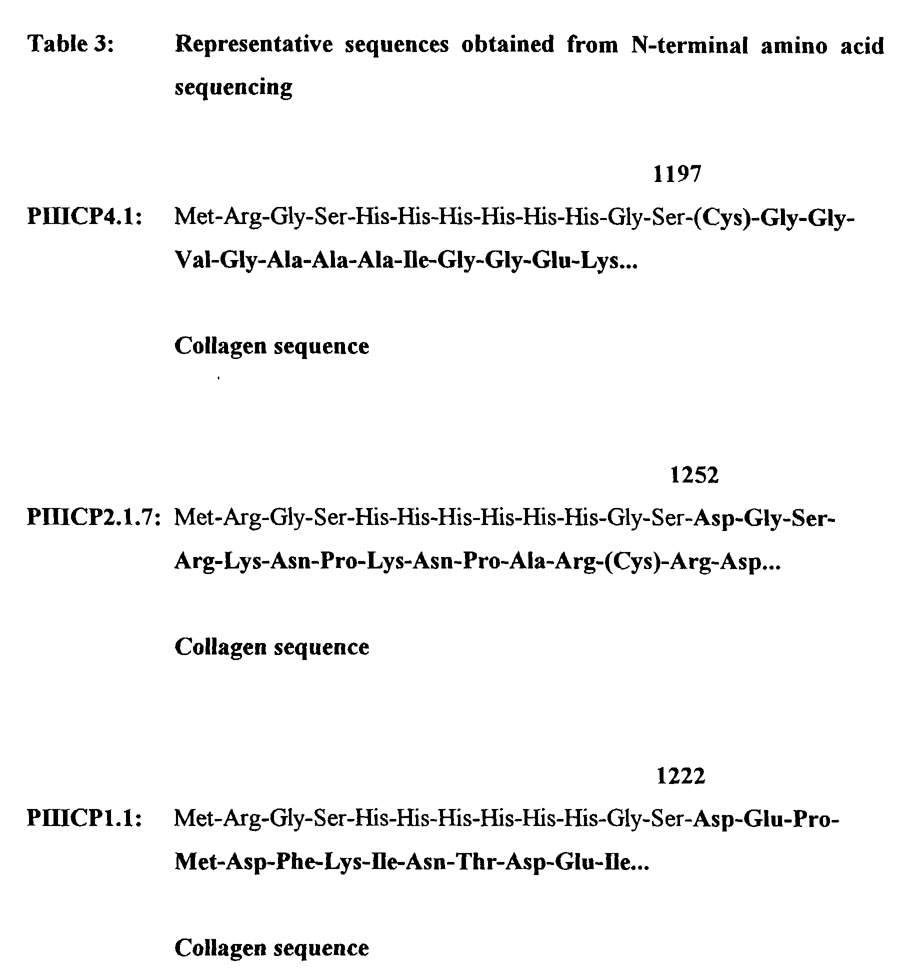

**Table 4**

| **Summary of the reactivity of the epitope-specific monoclonal antibodies against the recombinant PIIICP proteins** | | |
|---|---|---|
| Antigen | Antibody 48B14 directed against N-terminal epitope [Reactivity] | Antibody 48D19 directed against C-terminal epitope [Reactivity] |
| PIIICP4.1 (intact PIIICP with 6xHis tag) | +++ | +++ |
| 4.5.2 (unrelated antigen with identical His tag sequence) | - | - |
| PIIICP1.1 (38 aa C-terminal deletion protein) | +++ | - |
| PIIICP2.1.7 (30 aa N-terminal deletion protein) | +++ | +++ |
| PIIICP5.1 (130 aa C-terminal deletion protein) | +++ | - |
| PIIICP6.1 (90 aa N-terminal deletion protein) | - | +++ |

Table 4 summarizes the results from ELISA assays measuring the reactivity of the two monoclonal antibodies against recombinant PIIICP4.1 and against the deletion mutant proteins. The N-terminal amino acid sequences were identical in all cases up to the beginning of the collagen sequences. The control protein, 4.5.2, was an N-terminal 6xHis tag fusion protein identical to the PIIICP proteins in its N-terminal sequence and was used to ascertain that the antibodies were not directed against the His tag sequence.

## Claims

1. An immunoassay for procollagen-III-C-terminal propeptide (PIIICP).

2. Antibodies specifically binding in region I of PIIICP.

3. Antibodies specifically binding in region II of PIIICP.

4. Antibodies according to claim 2 and 3 which are monoclonal.

5. Recombinant PIIICP.

6. Use of the antibodies according to claims 2 to 4 for the determination of PIIICP concentrations.


SEQUENCE LISTING

(1) GENERAL INFORMATION:

(i) APPLICANT:
(A) NAME: Bayer AG
(B) STREET: Bayerwerk
(C) CITY: Leverkusen
(E) COUNTRY: Germany
(F) POSTAL CODE (ZIP): 51368
(G) TELEPHONE: 02143061455
(H) TELEFAX: 0214303482

(ii) TITLE OF INVENTION: An immunoassay for procollagen-III-C-terminal
propeptide

(iii) NUMBER OF SEQUENCES: 14

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97119018.6

(2) INFORMATION FOR SEQ ID NO: 1:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 1:

CGCGGGATCC TGTGGTGGTG TTGGAGCCGC T 31

(2) INFORMATION FOR SEQ ID NO: 2:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 2:

CGCGAAGCTT TAAAAAGCAA ACAGGGCCAA CGT 33

(2) INFORMATION FOR SEQ ID NO: 3:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 3:

CGCGGGATCC GATGAACCAA TGGATTTCAA A 31

(2) INFORMATION FOR SEQ ID NO: 4:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 4:

CGCGAAGCTT GACTGTTTTG CTCCATTC                                    28

(2) INFORMATION FOR SEQ ID NO: 5:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 5:

CGCGGGATCC GATGGTTCTC GTAAAAACCC                                    30

(2) INFORMATION FOR SEQ ID NO: 6:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 6:

CGCGAAGCTT TAAAAAGCAA ACAGGGCCAA CGT                                    33

(2) INFORMATION FOR SEQ ID NO: 7:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Protein

(xi) SEQUENCE DESCRIPTION: SEQ ID NO: 7:

CGCGGGATCC GATGAACCAA TGGATTTCAA A 31

(2) INFORMATION FOR SEQ ID NO: 8:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 8:

CGCGAAGCTT AAAACCACCA TCCATGGACT C 31

(2) INFORMATION FOR SEQ ID NO: 9:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 9:

CGCGGGATCC TGCATAAGTG CCAATCCTTT G 31

(2) INFORMATION FOR SEQ ID NO: 10:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Primer

(xi) SEQUENCE DESCRIPTION: SEQ ID NO: 10:

CGCGAAGCTT TAAAAAGCAA ACAGGGCCAA CGT 33

(2) INFORMATION FOR SEQ ID NO: 11:

(i) SEQUENCE CHARACTERISTICS:
(A) LENGTH: 25 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(vi) ORIGINAL SOURCE:
(A) ORGANISM: Collagen

(xi) SEQUENCE DESCRIPTION: SEQ ID NO: 11:

Met Arg Gly Ser His His His His His His Gly Ser Cys Gly Gly Val
1               5                   10                  15

Gly Ala Ala Ala Ile Gly Gly Glu Lys
            20                  25

(2) INFORMATION FOR SEQ ID NO: 12:

(i) SEQUENCE CHARACTERISTICS:
(A) LENGTH: 27 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(vi) ORIGINAL SOURCE:
(A) ORGANISM: Collagen

(xi) SEQUENCE DESCRIPTION: SEQ ID NO: 12:

Met Arg Gly Ser His His His His His His Gly Ser Asp Gly Ser Arg
1               5                   10                  15

Lys Asn Pro Lys Asn Pro Ala Arg Cys Arg Asp
            20                  25

(2) INFORMATION FOR SEQ ID NO: 13:

(i) SEQUENCE CHARACTERISTICS:
(A) LENGTH: 25 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(vi) ORIGINAL SOURCE:
(A) ORGANISM: Collagen

(xi) SEQUENCE DESCRIPTION: SEQ ID NO: 13:

Met Arg Gly Ser His His His His His His Gly Ser Asp Glu Pro Met
1               5                   10                  15

Asp Phe Lys Ile Asn Thr Asp Glu Ile
            20                  25

(2) INFORMATION FOR SEQ ID NO: 14:

(i) SEQUENCE CHARACTERISTICS:
(A) LENGTH: 270 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(vi) ORIGINAL SOURCE:

(A) ORGANISM: procollagen

(xi) SEQUENCE DESCRIPTION: SEQ ID NO: 14:

Cys Gly Gly Val Gly Ala Ala Ala Ile Ala Gly Ile Gly Gly Glu Lys
1               5                   10                  15

Ala Gly Gly Phe Ala Pro Tyr Tyr Gly Asp Glu Pro Met Asp Phe Lys
            20                  25                  30

Ile Asn Thr Asp Glu Ile Met Thr Ser Leu Lys Ser Val Asn Gly Gln
        35                  40                  45

Ile Glu Ser Leu Ile Ser Pro Asp Gly Ser Arg Lys Asn Pro Ala Arg
    50                  55                  60

Asn Cys Arg Asp Leu Lys Phe Cys His Pro Glu Leu Lys Ser Gly Glu
65                  70                  75                  80

Tyr Trp Val Asp Pro Asn Gln Gly Cys Lys Leu Asp Ala Ile Lys Val
                85                  90                  95

Phe Cys Asn Met Glu Thr Gly Glu Thr Cys Ile Ser Ala Asn Pro Leu
            100                 105                 110

Asn Val Pro Arg Lys His Trp Trp Thr Asp Ser Ser Ala Glu Lys Lys
        115                 120                 125

His Val Trp Phe Gly Glu Ser Met Asp Gly Gly Phe Gln Phe Ser Tyr
    130                 135                 140

Gly Asn Pro Glu Leu Pro Glu Asp Val Leu Asp Val Gln Leu Ala Phe
145                 150                 155                 160

Leu Arg Leu Leu Ser Ser Arg Ala Ser Gln Asn Ile Thr Tyr His Cys
                165                 170                 175

Lys Asn Ser Ile Ala Tyr Met Asp Gln Ala Ser Gly Asn Val Lys Lys
            180                 185                 190

Ala Leu Lys Leu Met Gly Ser Asn Glu Gly Glu Phe Lys Ala Glu Gly
        195                 200                 205

Asn Ser Lys Phe Thr Tyr Thr Val Leu Glu Asp Gly Cys Thr Lys His
    210                 215                 220

Thr Gly Glu Trp Ser Lys Thr Val Phe Glu Tyr Arg Thr Arg Lys Ala
225                 230                 235                 240

Val Arg Leu Pro Ile Val Asp Ile Ala Pro Tyr Asp Ile Gly Gly Pro
                245                 250                 255

Asp Gln Glu Phe Gly Val Asp Val Gly Pro Val Cys Phe Leu
            260                 265                 270